# EUROPEAN PATENT SPECIFICATION

(11) **EP 0 765 166 B1**
(45) Date of publication and mention of the grant of the patent: **21.05.2003**
(21) Application number: 95923885.8
(22) Date of filing: 13.06.1995
(51) Int. Cl.: A61K 51/08, C07K 14/47

(54) **RADIOLABELED ANNEXIN-GALACTOSE CONJUGATES**
RADIOAKTIVMARKIERTE ANNEXIN-GALAKTOSE KONJUGATE
CONJUGUES RADIOMARQUES D'ANNEXINE-GALACTOSE

(30) Priority: 16.06.1994 US 261064
(43) Date of publication of application: 02.04.1997
(73) Proprietor: NEORX CORPORATION, Seattle Washington 98119 (US)
(72) Inventor: RENO, John M., Brier, WA 98036 (US); KASINA, Sudhakar, Mercer Island, WA 98040 (US)
(74) Representative: Gowshall, Jonathan Vallance
(86) International application number: US9507599
(87) International publication number: WO95034315

(56) References cited:
- WO-A-89/10140
- WO-A-91/07187
- WO-A-92/19279
- WO-A-94/19024
- US-A- 4 897 255
- US-A- 5 371 184
- US-A- 5 380 646
- US-A- 5 627 036
- SARKAR H S ET AL: "Syntheses of Several Tc and I Labeled Neoglycoalbumins and Their Differential Uptake Patterns in Animal Biodistribution Experiments" NUCLEAR MEDICINE AND BIOLOGY, vol. 5, no. 22, July 1995, page 589-597 XP004051756
- STRATTON JR ET AL: "Selective uptake of radiolabeled annexin V on acute porcine left atrial thrombi." CIRCULATION, NOV 15 1995, 92 (10) P3113-21, UNITED STATES, XP002058828
- KASINA S ET AL: "Preformed chelate Tc-99m radiolabeling of r-annexin V for arterial thrombus imaging" JOURNAL OF NUCLEAR MEDICINE, 37 (5 SUPPL.). 1996. 29P., XP002058829
- BRITISH JOURNAL OF CANCER, Volume 61, issued 1990, SHARMA et al., "Inactivation and Clearance of an Anti-Cea Carboxypeptidase GS Conjugate in Blood After Localisation in a Xenograft Model", pages 659-662.
- THE JOURNAL OF NUCLEAR MEDICINE AND APPLIED SCIENCES, Volume 32, Number 2, issued 1988, GALLI et al., "A Radiopharmaceutical for the Study of the Liver: 99MTC-DTPA--Asialo-Orosomucoid", pages 110-116.
- BIOCHEMISTRY, Volume 15, Number 18, issued 1976, KRANTZ et al., "Attachment of Thioglycosides to Proteins: Enhancement of Liver Membrane Binding", pages 3963-3968.
- LEE C. Y. ET AL: '2-Imino-2-methoxyethyl 1-Thioglycosides: New Reagents for Attaching Sugars to Proteins' BIOCHEMISTRY vol. 15, no. 18, 1976, pages 3956 - 3962

## Description

### Cross-Reference to Related Applications

This application is a continuation-in-part of pending U.S. Patent Application No. 08/185,660, filed January 24, 1994.

### Technical Field

The present invention is directed to radiolabeled annexin-galactose conjugates. Also contemplated by the present invention are imaging protocols which involve the administration of a radiolabeled annexin-galactose conjugates. The annexin component of the conjugate serves to deliver the radiolabeled active component of the conjugate to vascular thrombi target sites. The galactose component of the conjugate facilitates rapid elimination of the radiolabeled annexin-galactose conjugate from the circulation of a recipient.

### Background of the Invention

When patients present with chest pain, palpitations or any other symptoms of coronary trauma or disease, the presence of vascular thrombi in the heart is a potential significant complicating factor for treatment. If a medical practitioner could non-invasively determine whether one or more vascular thrombi were present and, if present, the location of those vascular thrombi, better evaluation of treatment options would be possible. Furthermore, if a medical practitioner could determine that no vascular thrombi were present, thereby eliminating a potential complication in treatment, cardiac conditions could be treated more safely and effectively.

Most present techniques for determining the presence of vascular thrombi are invasive and/or cumbersome, and/or fail to detect such thrombi with good sensitivity and specificity. Thus, an imaging agent useful for non-invasive vascular thrombi imaging is desirable. .

Annexins are a class of proteins that are characterized by calcium-mediated binding to anionic phospholipids. Anionic phospholipids are about 20-fold more highly associated with activated platelets than quiescent platelets, and activated platelets are associated with vascular thrombi.

Iodinated annexin V has been shown to localize to vascular thrombi in vivo, but has suboptimal imaging characteristics, possibly due to the pronounced beta phase of blood clearance owing to possible transiodination and/or metabolic degradation with reincorporation into serum macromolecules or non-target tissues. Free radioactive iodine or iodine-containing metabolic degradation products exposed non-target tissues, especially the thyroid gland, to radioactivity. In addition, the iodine radiolabel used is difficult to obtain and is not therefore practical for wide spread use. Consequently, improved radiolabeled annexin compounds are desirable.

### Summary of the Invention

The present invention provides radiolabeled annexin-hexose conjugates and methods of making and using the same. Preferred annexin-containing conjugates of the present invention are those suitable for radiolabeling with a diagnostic imaging agent including:
an annexin;
one or more hexose residues; and
an N₂S₂ or N₃S chelate associated with the annexin.

Also provided by the present invention are radiolabeled annexin-hexose conjugates suitable for imaging vascular thrombi, which radiolabeled annexin-hexose conjugates include:
an annexin;
one or more hexose residues;
an N₂S₂ or N₃S chelate associated with the annexin; and
a diagnostic radionuclide complexed by the chelate.

A preferred annexin for use in the present invention is annexin V. A preferred radiolabeled annexin-hexose conjugate for use in the present invention is characterized by the following structure: 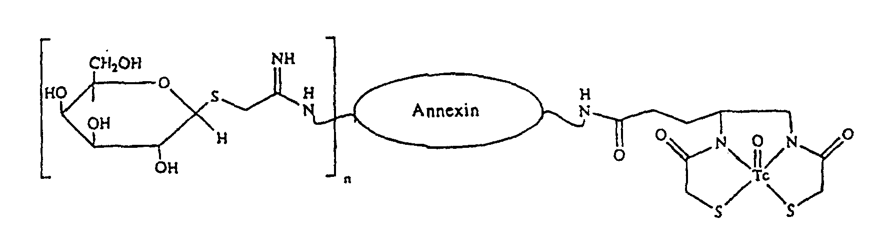 wherein n is 1 or more.

Preferred diagnostic radionuclides for use in the practice of the present invention are Tc-99m, Re-186 and Re-188, with Tc-99m being especially preferred. Vascular thrombi located in or near the heart are especially amenable to imaging in accordance with the present invention.

### Brief Description of the Drawings

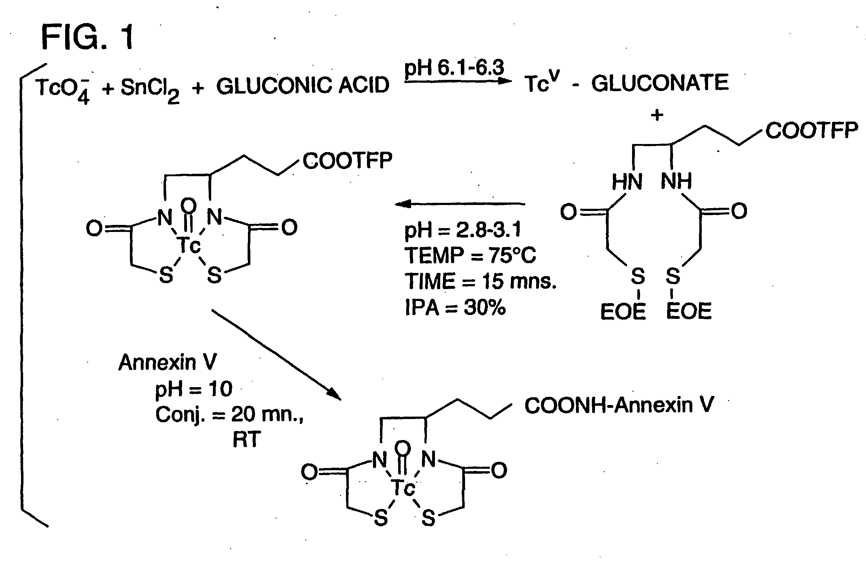

Fig. 1 schematically represents a method of radiolabeling annexin V.
Fig. 2 shows the blood clearance of Tc-99m-annexin V (o) and of Tc-99m-annexin V-galactose Δ).

### Detailed Description of the Invention

Prior to setting forth the invention, it may be helpful to set forth definitions of certain terms to be used within the disclosure.

Annexin: A class of compounds characterized by the ability to bind with high affinity to membrane lipids in the presence of millimolar concentrations of calcium. Annexins have been shown to exhibit anti-coagulatory effects that are mediated by the binding of annexins to negatively charged surface phospholipids (e.g., on activated platelets). This annexin-phospholipid binding is believed to block the activation of clotting factors by such negatively charged surface phospholipids. Prior to the recognition of the annexin class of molecules, members thereof were also referred to in the literature as placental anticoagulant proteins (e.g., PAP-1, 2, 3 and 4), lipocortins, calpactins, vascular coagulant (alpha and beta), calphobindin I, placental protein 4 (PP4), endonexin II, anchorin CII, calcium-dependent phospholipid binding protein, and the like. See Crumpton et al., Nature 345:212, 1990. Annexin-V is a prototypical annexin molecule used in the description of the present invention.

NₓS_{y} Chelates: As defined herein, the term "NₓS_{y} chelates" includes bifunctional chelators that are capable of (i) coordinately binding a metal or radiometal and (ii) covalently attaching to an annexin molecule. Particularly preferred NₓS_{y} chelates have N₂S₂ and N₃S cores. Exemplary NₓS_{y} chelates are described in Fritzberg et al., Proc. Natl. Acad. Sci. USA 85:4024-29, 1988; in Weber et al., Bioconj. Chem. 1:431-37, 1990; and in the references cited therein, for instance. For the purpose of this description, the prototypical NₓS_{y} chelate is an N₂S₂ chelate.

N₂S₂ Chelates: Diamide, dimercaptide bifunctional chelators of the NₓS_{y} family capable of stably complexing a radionuclide through two nitrogen atoms and two sulfur atoms that are appropriately positioned. Preferred N₂S₂ chelates are described in U.S. Patent No. 4,897,225, for example.

N₃S Chelates: Triamide, mercaptide bifunctional chelators of the NₓS_{y} family capable of stably complexing a radionuclide through three nitrogen atoms and one sulfur atom that are appropriately positioned. Preferred N₃S chelates are described in U.S. Patent No. 4,965,392, for example.

Radiolabeled Annexin: An annexin conjugated to a chelate having a radionuclide complexed therein.

Radiolabeled Annexin-Galactose: A galactose-derivatized annexin conjugated to a chelate having a radionuclide complexed therein.

Conjugate: A conjugate encompasses chemical conjugates (covalently or non-covalently bound), fusion proteins.

The present invention is directed to annexin-containing conjugates, radiolabeled annexin-galactose conjugates and the use thereof for diagnostic imaging purposes. Radiolabeled annexins are characterized by the following: rapid accretion to target cell sites characterized by anionic phospholipids; short circulating half-life; in vivo stability against metabolic degradation or radionuclide separation from chelate; and amenability to packaging in cold kit format.

Radiolabeled annexin-hexose conjugates of the present invention also exhibit the aforementioned characteristics, albeit to different degrees. For example, radiolabeled annexin-galactose conjugates exhibit a shorter circulating half-life than their radiolabeled annexin counterparts. Also, radiolabeled annexin-galactose conjugates generally exhibit a lower binding affinity for target sites than their radiolabeled annexin counterparts. Successful diagnostic imaging involves both target site accumulation of signal and rapid elimination of non-targeted signal. Consequently, the enhanced' elimination from a recipient's circulation of the radiolabeled-annexin-galactose conjugates of the present invention provides a distinct opportunity to achieve diagnostic images in a shorter time frame.

There are disclosed annexin-containing conjugates suitable for radiolabeling with a diagnostic imaging agent including:
an annexin;
one or more galactose residues; and
an NₓS_{y} chelate associated with the annexin.
Radiolabeled annexin-galactose conjugates suitable for imaging vascular thrombi anywhere in the recipient (but particularly in or near the heart) are also disclosed, which radiolabeled annexin-galactose conjugates incorporate an annexin, one or more galactose residues, an NₓS_{y} chelate, and further a diagnostic radionuclide complexed by the chelate.

The present invention involves annexin-containing conjugates suitable for radiolabeling with a diagnostic imaging agent including:
an annexin;
one or more hexose residues; and
an N₂S₂ or N₃S chelate associated with the annexin.
Radiolabeled annexin-galactose conjugates suitable for imaging vascular thrombi are also contemplated, which radiolabeled annexin-galactose conjugates incorporate an annexin, one or more galactose residues, an N₂S₂ chelate, and further a diagnostic radionuclide complexed by the chelate.

For the visualization of vascular thrombi associated with a number of pathological conditions, a conjugate of an annexin with a chelate complexed with an imaging radionuclide, such as Tc-99m for example, is administered to a recipient for whom such a diagnosis is desired. The annexin portion of the conjugate localizes rapidly to target sites characterized by negatively charged surface phospholipids, such as vascular thrombi. The radionuclide is selected for its ability to be visualized via one of the various techniques therefor, e.g., gamma camera imaging. Because of the rapid accretion of annexins to the target site and the short serum half-life (generally less than 30 minutes) of annexins (which is not significantly lengthened upon radiolabeling), imaging of those target sites proceeds with little exposure of non-target sites to radioactivity.

Diagnostic imaging is dependent on signal-to-noise ratio. Improvements involving either target signal accumulation or noise reduction will enhance the efficacy of the diagnostic imaging product. For targeted imaging, noise reduction is synonymous with reducing background radioactivity, particularly blood pool activity. Annexins, including annexin V, are rapidly removed by the liver; however, only a fraction of the activity is removed with each pass of the circulating conjugate through the liver.

To more efficiently remove the background activity from the blood, an improvement in liver extraction of annexin-containing conjugates is employed. The method to enhance liver extraction involves derivatizing the annexin-containing conjugate with moiety, such as galactose, that is recognized by a liver receptor. The efficiency of liver receptor extraction of the moiety recognized thereby will result in increased "per pass" removal by the liver of derivatized annexin-containing conjugate (e.g., annexin-galactose conjugate) in comparison to the amount of underivatized annexin-containing conjugate so removed.

Annexins are generally (with the most notable exception being annexin II) single chain, non-glycosylated proteins of approximately 33-72 kilodalton molecular weight. Annexins possess a number of biological activities associated with calcium ion-mediated binding.

Investigations have shown that annexins bind with high affinity to anionic membrane lipids in the presence of millimolar concentrations of calcium. In the presence of calcium, these proteins have an especially high affinity for negatively charged phospholipids, such as phosphatidylserine, phosphatidylglycerol, phosphatidic acid, or phosphatidylinositol. See, for example, Funakoshi et al., Biochem. 26: 5572-78, 1987; and Tait et al., Biochem. 27: 6268-76, 1988. Such negatively charged phospholipids are associated with vascular thrombi (e.g., are located on the surface of activated human platelets).

Annexins exert anti-coagulatory effects. Coagulation inhibition is mediated by the binding of annexins to negatively charged surface phospholipids (e.g., present on the surface of activated platelets). This binding is believed to block the activation of clotting factors by such negatively charged surface phospholipids. Annexins localize to target sites bearing anionic phospholipids rapidly, i.e., in a matter of approximately 5 to 30 minutes depending on circulating levels thereof, but remain circulating in the serum for a somewhat longer time period (circulating half-life < 30 minutes). Example III below discusses results of imaging experiments wherein vascular thrombi were visualized in planar images at an average time (following annexin administration) of 82 minutes.

Because of these properties, annexins or annexins conjugated to diagnostic or therapeutic agents may be employed in protocols for the diagnosis or treatment of vascular thrombi associated with a number of indications, such as DVT (deep vein thrombosis), PE (pulmonary embolism), myocardial infarction, atrial fibrillation, problems with prosthetic cardiovascular materials, stroke . Exemplary diagnostic protocols and experimentation employing radiolabeled annexins are set forth below to further elucidate this aspect of the present invention.

An example of a preferred annexin useful in the practice of the present invention is Annexin V, which was isolated by Bohn in 1979 from human placenta, a rich source of annexins, and termed Placenta Protein 4 (PP4). Annexin V has been expressed in E. coli. Also, a full length cDNA clone of annexin V has been obtained and subcloned in expression vectors, thereby facilitating the production of fusion proteins containing annexin V. Annexin V consists of four domains (four tandem, imperfect repeats of about 75 amino acid residues, Funakoshi et al., Biochem. 26: 8087-92, 1987), wherein each domain is made up of 5 alpha helices. From the side, the annexin V molecule appears crown-like with at least four calcium binding sites on its convex surface, through which annexin-phospholipid interactions are mediated. Other annexin molecules are also useful in the practice of the present invention, and the discussions relating to annexin V herein apply generally to annexin molecules.

Because annexin V has a plurality of calcium binding sites, and because annexin V binding to negatively charged phospholipids is mediated by calcium, an engineered molecule consisting of one or more individual annexin V domains may be employed in imaging protocols of the present invention. Also, the annexin molecule may be partitioned at a position or positions different from the domain boundaries to provide an engineered molecule capable of calcium-mediated binding of anionic phospholipids. Also, annexin V may be altered at one or more amino acid residues, so long as the affinity of annexin V for anionic phospholipids is not significantly impaired. The degree of annexin binding to phospholipids may be quantified by fluorescence quenching as described by Tait et al., J. Biol. Chem. 264: 7944-49, 1989.

Among annexins, annexin V has the strongest binding affinity (K_{d} < 10⁻¹⁰ M) for phospholipid vesicles containing 80% phosphatidylcholine and 20% phosphatidylserine under conditions comparable to plasma and extracellular fluid (1.2 mM ionized calcium, 0.15 M ionic strength). This binding is reversible and calcium dependent.

To decrease the background radiolabel activity, annexins may be derivatized with hexose or hexose-based moieties. More specifically, annexins may be derivatized to incorporate one or more hexoses (six carbon sugar moieties) recognized by Ashwell receptors or other liver receptors, such as the mannose/N-acetylglucosamine receptor, which are associated with endothelial cells and/or Kupffer cells of the liver, or by the mannose 6-phosphate receptor. Exemplary of such hexoses and hexose-based moieties are galactose, mannose, mannose 6-phosphate, N-acetylglucosamine, pentamannosyl phosphate. Other moieties recognized by Ashwell receptors, including glucose, N-galactosamine, N-acetylgalactosamine, thioglycosides of galactose and, generally, D-galactosides and glucosides , may also be used in the practice of the present invention.

Galactose is the prototypical hexose employed for the purposes of this description. Galactose thioglycoside conjugation to a protein is preferably accomplished in accordance with the teachings of Lee et al., "2-Imino-2-methoxyethyl 1-Thioglycosides: New Reagents for Attaching Sugars to Proteins," Biochemistry 15(18): 3956, 1976. Another useful galactose thioglycoside conjugation method is set forth in Drantz et al, "Attachment of Thioglycosides to Proteins: Enhancement of Liver Membrane Binding," Biochemistry 15(18): 3963, 1976. Annexin-galactose conjugation is also discussed in the Examples set forth herein.

Hexose conjugation to the annexin via chemical methods can occur either prior to (post-formed approach) or following (pre-formed approach) conjugation of the chelate to the annexin. Hexose chemical conjugation is preferably conducted prior to chelate conjugation, however.

The number of galactose residues on the product conjugates will range from 1 to the maximum number of galactoses that do not significantly diminish the binding affinity of annexin for its target. For example, galactose derivatization that preserves at least 20% of native annexin binding activity is preferred, with the preservation of at least 50% of native annexin binding activity more preferred. The theoretically possible maximum number of galactose residues located on the annexin molecule is 22 (i.e., the number of lysine residues within the annexin structure). An exemplary number of galactose residues on radiolabeled annexin-galactose conjugates of the present invention ranges between 1 and 5.

Annexin V is radiolabeled with an imaging radionuclide for use in the present invention. Radionuclides useful within the present invention include gamma-emitters, positron-emitters, Auger electron-emitters, X-ray emitters, fluorescence-emitters Radionuclides suitable for use in the present invention are known in the art and include ⁶⁴Cu, ¹⁸⁶Re, ¹⁸⁸Re, ¹⁰⁰Pd, ²¹²Bi, ²¹²Pb, ¹⁰⁹Pd, ⁶⁷Cu, ^{99m}Tc, ⁹⁴Tc, ⁹⁵Ru, ¹⁰⁵Ru, ⁹⁹Rh, ¹⁰⁵Rh, ¹¹¹In, ¹⁵³Sm, ¹⁷⁷Lu, ¹⁷⁰Lu, ¹⁸⁹Pt, ¹⁹³Pt, ¹⁹⁹Au, ¹⁹⁷Hg.

Tc-99m is a preferred radionuclide for the practice of the present invention. Tc-99m has been stably bound to annexin V in accordance with the present invention at both low and high specific activity (0.53 µCi/µg - 101.2 µCi/µg). Adequate radiochemical yields and good radiochemical purities were obtained. Activated platelet binding studies were also conducted, and the radiolabeled annexin V conjugates bound to activated platelets well.

N₂S₂ and N₃S chelates are known in the art. For example, preferred N₂S₂ chelates are described in U.S. Patent No. 4,897,225, and preferred N₃S chelates are described in U.S. Patent No. 4,965,392. The present inventors have applied this stable chelation technology to exploit the thrombus-targeting ability of annexin molecules, thereby providing an imaging agent which is able to rapidly visualize vascular thrombi in vivo. The radiolabeled annexins and radiolabeled annexin-galactose conjugates of the present invention can be used to obtain thrombus images which reduce or eliminate the high level of background radioactivity that results from metabolically degraded radiolabeled conjugate. The radiolabeled annexins and radiolabeled annexin-galactose conjugates also avoid clinically unacceptable toxicity to non-target cell sites. Tc-99m radiolabeled annexin V performed better than I-123 in the pig studies set forth in Example III hereof.

Radiolabeling of annexin V with a radionuclide using an N₂S₂ or N₃S chelate may be conducted using either a pre-formed or a post-formed approach. That is, the radionuclide is either complexed within the chelate prior to (pre-formed) or following (post-formed) conjugation of the chelate to annexin V. The pre-formed approach is preferred and suitable procedures therefor are set forth in Examples I, II, and IV.

Annexin molecules may be modified by the addition of from 2 to 6 terminal amino acid residues to facilitate the conjugation reaction between the annexin molecule and the chelate or between the annexin molecule and a galactose residue. For example, terminal amino acid residues may be added to provide a sulfhydryl group or to provide a group capable of derivatization to a maleimide group, with such sulfhydryl and maleimide groups available for the conjugation reaction. This modification may be made by protein chemistry methods or via production of an appropriate fusion protein or other techniques useful therefor.

Radiolabeled annexins and radiolabeled annexin-galactose conjugates of the present invention offer an additional advantage over the previously prepared I-123-labeled annexins, in that they are amenable to packaging in a cold kit. That is, the annexin or annexin-galactose and chelate components may be individually vialed and provided separately from the Tc-99m component (and, possibly, vialed separately from each other). When a patient requiring a thrombus image is identified, a cold kit may be ordered or retrieved from storage; Tc-99m may be obtained from a radiopharmacy or other source thereof; the pre-formed or post-formed chelation/complexation process is performed; the radiolabeled annexin or radiolabeled annexin-galactose conjugate is administered to the patient; and the patient is subsequently imaged. For radiolabeled annexin-galactose conjugates, an annexin-galactose conjugate is preferably prepared and vialed in the kit.

Lyophilization and vialing in a sterile, pyrogen-free environment of the conjugate components may be accomplished via techniques, known to persons skilled in the art of good manufacturing actices, particularly as such practices relate to biological materials.

Radiolabled annexin-hexose conjugates of the present invention are administered in such amounts as to deliver a diagnostically effective amount of radionuclide to target sites. Appropriate administered doses depend on a variety of factors that are largely patient specific, and practitioners in the art will consider such factors in the practice of the present invention. The components of the radiolabeled annexin-hexose conjugates also impact dose amounts in ways that are known to or routinely ascertainable by practitioners in the art. In general, radiolabeled annexin or radiolabeled annexin-galactose conjugate is administered to large mammals at a dose ranging between 0.3 and 300 µg/kg body weight of the recipient, with from 3 to 10 µg/kg preferred, depending upon the physiological characteristics of the patient and the ailment involved or suspected. A practitioner in the art is capable of identifying an appropriate dose and administration route for a given recipient with a given ailment.

Radiolabeled annexin-hexose conjugates of the present invention may be administered in any convenient manner therefor. For example, intravenous infusion may be employed to administer radiolabeled annexins or radiolabeled annexin-galactose conjugates. Other routes of administration also find utility in the practice of the present invention. Exemplary additional administration routes are injection by the arterial (e.g., coronary artery), intracoronary, intralymphatic, intrathecal, or other intracavity routes. .

After administration of the radionuclide, depending upon the nature of the radionuclide and the purpose of the administration, the recipient may be subject to various procedures for detection of radioactive emissions from the site or sites at which the radionuclide localizes. For example, conjugates containing Tc-99m are imageable by a gamma camera.

The invention is further described through presentation of the following examples. These examples are offered by way of illustration.

### Comparative Example I

### Procedure for Radiolabeling an Annexin - N₂S₂ Chelate Conjugate

Annexin V can be isolated from a variety of tissue extracts, such as liver, lung and placenta, in accordance with procedures set forth in Funakoshi et al., Biochem. 26: 8087-92, 1987); Tait et al., Biochem. 27: 6268-76, 1988; and U.S. Patent No. 4,937,324, for example. In addition, annexin V can be expressed in E. coli, as described by Tait et al., Archives of Biochemistry and Biophysics 288: 141-44, 1991.

Annexin V was radiolabeled with Tc-99m by using a diamide dimercaptide N₂S₂ chelate in accordance with the OncoTrac® Small Cell Lung Cancer Imaging Kit labeling procedure described in J. Nucl. Med. 32: 1445-51, 1991.

A preferred method for radiolabeling annexin V with Tc-99m constitutes a modified OncoTrac® kit procedure using C-18 Baker purified Tc-99m-N₂S₂-TFP. In this procedure, an acidified active ester solution was prepared by adding 0.16 ml of 0.2M hydrochloric acid: glacial acetic acid (14:2 ratio) to 0.6 ml of 2,3,5,6,-tetrafluorophenyl 4,5-bis-(S-1-ethoxy-ethylmercaptoacetamido)pentanoate (0.3 mg, 0.0005 mole freshly dissolved in 0.9 ml of isopropyl alcohol). Then 0.5 ml of this solution was added to 1.1 ml of Tc-99m-gluconate (prepared from 0.12 mg SnCl₂ 2 H₂O, 5.0 mg sodium gluconate at pH 6.1-6.3, and 100 mCi/ml of [Tc-99m] pertechnetate, i.e., the first step in the OncoTrac® kit labeling procedure). The reaction mixture was heated at 75°C for 15 minutes followed by cooling on ice. The resulting Tc-99m transchelated tetrafluorophenyl active ester derivative of Tc-99m-4,5-bis(thioacetamido)pentanoate was, optionally and preferably diluted with 2 ml water and purified by loading the reaction mixture on a conditioned C-18 cartridge (J.T. Baker), washing with 2.0 ml water eight times followed by drying the column for 5 minutes, and eluting with 100% acetonitrile. The solvent was evaporated under a steady stream of N₂. Then 0.15 ml of phosphate buffered saline (PBS), 0.15 ml of annexin V at 2.35 mg/ml, and 0.2 ml of 0.2 M bicarbonate (pH 10.0) were added for conjugation to Tc-99m-N₂S₂. After 20 minutes at room temperature, the Tc-99m-N₂S₂-annexin V conjugate was purified by passage through a G-25 SEPHADEX® (PD-10) column (available from Pharmacia) equilibrated with PBS. Fractions (1.0 ml) were collected, and those fractions containing annexin V were pooled. Protein concentration was determined by UV absorption at 280 nm. Tc-99m-annexin V (300 -350 mg) conjugate solution was diluted and stored in PBS containing bovine serum albumin (BSA) at a final concentration of 15-20 mg BSA/ml PBS prior to injection.

### Comparative Example II

### Procedure for Radiolabeling an Annexin - N₃S Chelate Conjugate

S-benzoylmercaptoacetylglycylglycylglycine (S-benzoyl MAG₃) is prepared in accordance with the procedures described in U.S. Patent No. 4,965,392. Then 25 micrograms of S-benzoylmercaptoacetylglycylglycylglycine is dissolved in 0.10 ml of 1.10 M carbonate buffer (pH 12). Then 75 mCi of Tc-99m pertechnetate is added in about 1.0 ml followed by 1.0 mg of freshly dissolved sodium dithionite (10 mg/ml). This mixture is heated at 100°C, plus or minus 4°C, for 3 minutes, then is cooled in an ice bath for 5 minutes to give Tc-99m-MAG₃ as determined by ITLC (CH₃CN solvent); anion exchange HPLC (Beckman AX, 10 micron 0.01M Na₂SO₄/0.01M Na₃PO₄, pH 7.0); and reverse phase HPLC (Beckman ODS, 5 micron 2% CH₃CN/0.01M Na₃PO₄, pH 7.0).

The Tc-99m-MAG₃ complex in carboxylate form is then esterified; 0.20 ml 1N HCl, 0.30 ml of 0.2M phosphate buffer pH 6.0, 10.0 mg 2,3,5,6-tetrafluorophenol (TFP) in 0.01 ml 90% CH₃CN, and 12.5 mg of EDC (1-(3-dimethylaminopropyl)-3-ethylcarbodiimide hydrochloride) in 0.10 ml of 90% CH₃CN are combined, and the reactants are mixed at room temperature (25°C, plus or minus 2°C) for 1 hour. At this point, a yield of Tc-99m-MAG₃-TFP ester as determined by ITLC (CH₃CN solvent); anion exchange HPLC (Beckman AX, 10 micron 0.01M Na₂SO₄/0.01M Na₃PO₄, pH 7.0); and reverse phase HPLC (Beckman ODS, 5 micron 34% CH₃CN/0.01M Na₃PO₄, pH 7.0). The preparation is purified using a C-18 Baker column. The reaction mixture is, optionally and preferably diluted with 2 ml water and loaded in the column, washed two times with water and then eight times with 10% C₂H₅OH/0.01M Na₃PO₄, pH 7.0. The product is eluted with CH₃CN, and the solvent is removed prior to conjugation with annexin V.

The conjugation of the active ester to annexin V is carried out by adding annexin V in a phosphate buffer, pH 9.5, to the Tc-99m-MAG₃-TFP ester. The reaction is carried out for at least 30 minutes, and the desired radiolabeled annexin product is obtained by passage through a PD-10 gel filtration column.

### Comparative Example III

### Thrombus Imaging With a Radiolabeled Annexin

A. Animal Preparation - LA Vascular Thrombi. Fasting 25-30 kg Yorkshire swine were sedated with intramuscular Telazol (5-10 mg/kg) (commercially available from AVECO Co.) and commercially available Atropine (1 mg, Elkins-Sinn, Inc., Cherry Hill, NJ). Surital (200 mg) (commercially available from Abbott Laboratories) anaesthesia was administered intravenously. The animals were intubated and given inhalation anaesthesia of 1.5-2% halothane (commercially available from Abbott Laboratories) and O₂ sufficient to obtain a deep level of anesthesia and physiologic arterial blood gases. Continuous electrocardiographic monitoring was instituted using alligator clip electrodes. A cut down in the neck region was conducted, and an 8 french catheter (USCI Co, Billerica, MA) was placed in the right common carotid artery for blood pressure and arterial blood gas monitoring as well as for blood sampling.
   The swine were placed in a right lateral decubitus position, and a lateral thoracotomy was performed to expose the heart. The incision was held open by a thoracotomy retractor. The pericardium was opened, and the left atrial appendage was isolated from the left atrium by a vascular cross- clamp. Rubber tipped forceps were used to gently crush the appendage. Five minutes later, ricinoleate (1 mg, ICN Pharmaceuticals, Costa Mesa, CA) and thrombin (50 mg, Johnson and Johnson Co., Arlington, Texas) were injected into the LAA (left atrial appendage) using a 27 Ga needle. The cross-clamp was removed 10 minutes later.
   One hour after the crush injury, Tc-99m-annexin V, prepared in accordance with Example I above, was administered as an intravenous bolus dose in an ear vein. The intravenous line was then flushed with saline. In 7 animals, I-125 labeled ovalbumin was administered as a non-specific control preparable, for example, by the procedure described by Fraker et al., Biochem. Biophys. Res. Commun. 80: 849-57, 1978). Briefly, I-125-radiolabeled ovalbumin was prepared by the Iodogen method, employing 600 µg ovalbumin (Sigma Chemical Co., St. Louis, Missouri) and NaI-125 (2mCi, 0.92 nmol).
   Image acquisition was performed as described below. At the end of the experimental procedure (generally about 150 minutes), the animals were sacrificed by an intravenous bolus dose of 80 mEq of KCl while the animals were still under general anaesthetic. A final blood sample was taken for well counting. The heart was rapidly excised, washed free of blood and dissected into samples for well counting. Additional samples of carotid artery, lung, liver, spleen, muscle and kidney were obtained in some animals.
B. Controls. Five different types of controls were used: open chest sham; closed chest sham; ovalbumin; indium platelets; and non-specific Tc-99m-labeled antibody.
   1. Open Chest Sham. In three animals, the heart was exposed as above, but the left atrium was not isolated, crushed or injected with ricinoleate/thrombin. Marker images with a cobalt marker were performed as described below, and the Tc-99m-annexin V was injected 30-60 minutes after LAA exposure. Imaging and sample acquisition were identical to that described in A above.
   2. Closed Chest Sham. In seven animals, an ear intravenous line was established. No thoracotomy was performed. Sedation and anesthesia were identical to that described in A above. The Tc-99m-annexin V (+/other control radionuclides, such as I-125 ovalbumin or In-111-platelets) was administered and image acquisition was performed.
   3. Ovalbumin. I-125 ovalbumin was administered in 7 animals as a negative control protein. Ovalbumin has a molecular size similar to that of annexin, and exhibits a slightly slower blood clearance.
   4. Indium Platelets. In-111 platelet labeling was performed in 7 animals as a positive well counting label. In-111 radiolabeled platelets were prepared in accordance with the procedure described by Stratton et al., Am. J. Cardiol. 47: 874, 1981 and Stratton et al., Circulation 69: 561, 1984. Imaging was not attempted because of the long serum half-life of the In-111-platelets.
   5. Non-specific Tc-Labeled Antibody. In a single experiment, a left atrial (LA) thrombi was created by the above method, but Tc-99m-Annexin V was not administered. Instead, a Tc-99m-Fab fragment of an antibody designated as NR-LU-10 was administered. NR-LU-10 is a 150 kilodalton molecular weight IgG2b monoclonal antibody that recognizes an approximately 40 kilodalton glycoprotein antigen expressed on most carcinomas. NR-LU-10 is a well characterized pancarcinoma antibody that has been safely administered to over 565 patients in human clinical trials. NR-LU-10-Fab is prepared in accordance with known techniques and is radiolabeled in accordance with the procedures described in J. Nucl. Med. 32: 1445-51, 1991 and by the modified C-18 Baker purified Tc-99m-N₂S₂-TFP procedure described in Example I for preparing radiolabeled annexin V. This Tc-99m-Fab conjugate was designed as a negative control for both well counting and imaging.
C. Imaging. A cobalt marker was placed on the exposed surface of the LAA and held in place with surgical tape affixed to the thoracotomy retractor. The tape was adjusted so that the marker generally moved with the LAA with each cardiac cycle. Marker images were acquired for 10 seconds in each planar view and 10 seconds in each tomographic slice. The cobalt marker was then removed.
   A General Electric Starport camera with a general purpose collimator was used to acquire the Tc-99m images. Five minute planar acquisitions were performed sequentially in the left lateral, 45 LAO, and anterior views. These were followed by a 10 minute tomographic acquisition. This full set of 3 planar and 1 tomographic acquisitions was repeated for a total of 5 sets. Care was taken not to move the pig or imaging gantry during the entire imaging sequence.
   Images were recorded on a Microdelta computer slaved to a VAX mainframe system. Images were stored on tape or on the VAX hard drive. Planar image analysis consisted of first viewing the image with the marker and recording the marker position on the viewing terminal screen. The first image acquired after Tc-99m-annexin V injection was used to define the cardiac blood pool. Each subsequent image was viewed and analyzed using the marker and initial blood pool as references. Each image was scored as positive, equivocal or negative.
   Thirteen animals had left atrial thrombi created and were imaged as described above. Twelve animals had Tc-99m-annexin V injected for imaging, and one animal had a non-specific Tc-99m Fab injected as a control. Closed chest imaging was performed in 7 animals without atrial injury, and open chest sham experiments were performed in 3 animals. In animals with atrial thrombi, all planar images taken at less than 35 minutes after administration of Tc-99m-annexin V were negative. Nine of the atrial thrombi planar images taken at greater than 70 minutes were positive, 1 was equivocal and 2 were negative. All of the tomographic images of atrial thrombi were either positive (n=10) or equivocal (n=2) at imaging times of greater than 2 hours post injection. The average time in which the planar images became positive following administration of Tc-99m-annexin V was 82 minutes (35-135 minutes).
   None of the closed chest control animals'had positive images. One of the three open chest shams had a positive image at 85 minutes. This false positive is believed to result from the production of surgically-induced thrombi.
   These results indicate that intravenous administration of Tc-99m-annexin V permitted acquisition of diagnostic images identifying atrial vascular thrombi within a short time period following conjugate administration.
D. Sample Collection. Samples (both blood and tissue), as described above, were weighed and placed in vials for immediate counting of Tc-99m. After the Tc-99m had decayed (typically at 5-7 days), the samples were re-assayed to obtain the I-125 counts. Each vial was counted for 1 minute. The counts were corrected for decay, then for weight, and recorded as counts per minute per gram. The counts per minute per gram for each sample were then normalized by dividing the counts per minute per gram of the final blood specimen. Consequently, the results for each sample were calculated in a ratio with the last blood sample, permitting meaningful comparison between animals. This procedure was performed for all radionuclides in a given experiment.

Table I shows well counting ratios for a variety of tissue samples. For the injured tissues and thrombi, multiple specimens were usually taken from the same animal. In those cases, the maximum ratio for any one specimen is reported, as well as the average of all specimens taken. The maximum for each animal was averaged across all animals and is reported as the Maximum Anx-V Ratio.

These results indicate that Tc-99m-annexin V localizes preferentially to atrial thrombi and injured left atrium, with the highest non-target localization occurring in the kidney. The kidney level is at least partially indicative of excretion of Tc-99m-annexin V via the renal route.

### Example IV

### Radiolabeled Annexin-Galactose Conjugates

A. Annexin V -Preparation from human placenta and by expression in *E. coli*. Annexin V was purified from human placenta to a final purity of 99% as described by Funakoshi et. al., Biochemistry 26, 5572-78, 1987 and Tait et. al., Biochemistry 27, 6268-76, 1988.
Alternatively, annexin V was obtained, but not employed in the following experiments, by expression in *E. coli*. This expression was conducted as follows.
1. Preparation of bulk fermentation supernatant. Standard molecular biology techniques were used to express annexin V in *E. coli*. More specifically, the protein coding region of annexin V cDNA (see, Funakoshi et. al. referenced above) was inserted into the NdeI/BamHI site of plasmid pET-12a (Novagen, Madison WI). The selected plasmid was used to transform *E. coli* strain BL21(DE3) (Novagen).
   Individual colonies (clones) were isolated on Luria Broth plates containign 50 µg/ml ampicillin or carbenicillin, as discussed in Sambrook et al., "Molecular Cloning Laboratory Manual," 2nd ed., Cold Springs Harbor Laboratory Press, 1989. For production, cultures of the selected clones containing the desired plasmid were grown overnight at 37 °C with shaking in Terrific Broth (see, Tartof et. al., Bethesda Res. Lab. Focus, 9: 12, 1987) containing 50 µg/mL carbenicillin (commercially available from Sigma Chemical Co., St. Louis, Missouri). The culture was diluted 1:20 in the same medium and incubated at 37 °C with shaking for 18-24 hr. The bacteria were harvested by centrifugation, washed once with an equal volume of 0.05 M Tris-HCl, 0.15 M sodium chloride, pH 8, and stored as pellets at -20 °C. Pellets were thawed and resuspended with 10-15% (w/v) cold 0.05 M Tris-HCl, 0.02M Na₄EDTA, pH 7.2. The suspension was sonicated for 4 min on ice, centrifuged, and the supernatant stored at -70°C.
2. Purification of annexin V from bulk fermentation supernatant. After thawing, the supernatant was filtered using a 0.1 µm hollow fiber unit (commercially available from A/G Technology, Needham, MA), and the conductivity adjusted with concentrated sodium chloride to equal 0.02 M Tris-HCl, 0.5 M sodium chloride, pH 8. Polyethylene imine-WP (J.T. Baker, Phillipsberg, NJ) was suspended in 0.02 M Tris-HCl, 0.5 M sodium chloride, pH 8, and 1 g of resin per 10 ml of bulk fermentation supernatant was added to the filtered supernatant. After stirring for 30 min, the supernatant was removed, exchanged into 0.02 M Tris-HCl, pH 8, and applied to a Q-SEPHAROSE® HP column (Pharmacia, Piscataway, NJ) equilibrated in the same buffer. The column was developed with a step gradient of 0-0.5 M sodium chloride. Fractions were assayed for annexin V by size exclusion HPLC, and fractions containing A₂₈₀ absorbing material of appropriate size were pooled. The semi-purified annexin V was exchanged into PBS and concentrated by ultra-filtration. Gel filtration over SEPHACRYL® 100 HP (Pharmacia) equilibrated in PBS yielded the purified protein. The protein concentration was adjusted to 1 mg/ml, sterile 0.2 µm filtered, and stored at 2-8 °C.

B. Derivatization of Annexin V with Galactose. The general method of Lee et al., Biochemistry 15: 6268-76, 1976 was followed.
a. Preparation of 2-imino-2-methoxyethyl-1-thio-β-D-galactopyranoside.
   A 1.0 M solution of cyanomethyl-2,3,4,6-tetra-O-acetyl-1-thio-β-D-galactopyranoside (commercially available from Sigma Chemical Company, St. Louis, MO) was prepared by dissolving 1.83 g (4.5 mmole) in 4.5 ml anhydrous methanol with heating to 50 °C. The solution was maintained at 50 °C, and 0.1 ml (0.45 mmole) of 25% sodium methoxide in methanol (commercially available from Aldrich Chemical Company, Milwaukee, WI) was added with continuous stirring. After 6 hr at 50°C, the methanol was removed under reduced pressure yielding the galactose methyl imidate as a colorless, viscous oil.
b. Galactosylation of annexin V
   The galactose methyl imidate was offered to annexin V in molar ratios of 300:1, 150:1, and 75:1. For the 300:1 offering, 3.35 mg of galactose methyl imidate in methanol was taken to an oil under a stream of nitrogen. To the oil was added 2.53 mg of annexin V in 0.05 M HEPES buffer (commercially available from Sigma Chemical Co., St. Louis, Missouri), pH 7.4 and 0.04 ml of 0.5 M sodium borate buffer, pH 8.5. The solution was mixed for 1 hr at room temperature by tumbling the reaction vessel, and was then allowed to stand at room temperature overnight (approximately 18 hr). The reaction mixture was diluted with 0.4 ml PBS, transferred to dialysis tubing (6000-8000 MW cutoff), and dialyzed 24 hr against PBS. After removing the material from the dialysis bag, the solution was filtered through a 0.2 µm syringe filter. The other offering levels were conducted analogously.

C. Characterization of Galactose-Derivatized Annexin V. Protein concentration was determined using A₂₈₀ of 0.6 for a 1 mg/ml solution of annexin V. The number of galactose residues per molecule of annexin V was determined by measuring the total number of reactive amines on annexin V before and after reaction with galactose methyl imidate using trinitrobenzenesulfonic acid, as described by Habeeb, Analytical Biochemistry 14: 328-36, 1966.

| Offering Ratio | Substitution Ratio |
|---|---|
| 300:1 | 4.7:1 |
| 150:1 | 2.3:1 |
| 75:1 | 1.1:1 |

The ability of galactose-modified annexin V to bind to activated platelets was assessed by determining its ability to inhibit the binding of unmodified, ¹²⁵I radiolabeled annexin V to freshly isolated human platelets, following the method of Thiagarajan and Tait J. Biol. Chem. 265: 17240-43, 1990. The following table shows the results of the competition assay, both in absolute value (left column) and in a value normalized to 100% for unmodified annexin V (right column).

| Substitution Ratio | Competition (% of control) | Competition (% of control) |
|---|---|---|
| 4.7 | 0 | 0 |
| 2.3 | 5.7 | 7.6 |
| 1.1 | 21.4 | 28.7 |
| unmodified | 74.5 | 100 |

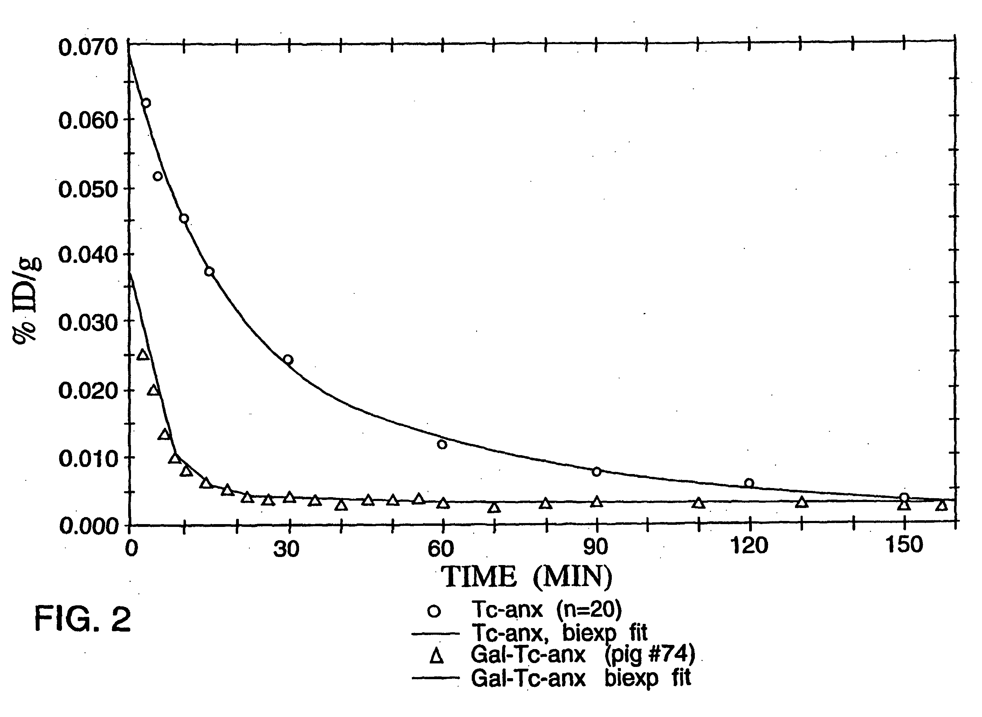

D. Preparation of Tc-99m-Annexin V-Galactose. Annexin V-galactose was radiolabeled with technetium-99m using a diamide dimercaptide (N₂S₂) chelate as described by Kasina et. al., J. Nucl. Med. 32: 1445-51, 1991. The preformed active ester chelate was diluted with 2.0 ml water and purified before conjugation to annexin V-galactose using a modified conditioned C-18 column (commercially available from J.T. Baker), as described by Fritzberg et. al., Proc. Natl. Acad. Sci. USA 85: 4025-29, 1988, washing with 2.0 ml water eight times followed by drying the column for 5 min and eluting with 100% acetonitrile. The solvent was removed under a steady stream of N₂. Then 0.15 ml of PBS, 0.35 ml of annexin V-galactose (4.7:1 galactose:annexin V) at 1.0 mg/ml, and 0.2 ml of 0.2M bicarbonate buffer (pH 10.0) were added for conjugation to Tc-99m-N₂S₂-TFP ester. After 20 min at room temperature, the Tc-99m-annexin V-galactose conjugate was purified by passage through a G-25 SEPHADEX® PD-10 column (commercially available from Pharmacia) equilibrated with PBS.
Fractions of 1.0 ml were collected, and fractions containing annexin V were pooled. Protein concentration was determined by UV absorption at 280 nm. The radiochemical yield of the conjugate was 35.3%. The radiochemical purity was 99.8%, as assessed by instant thin layer chromatography on silica gel impregnated glass fiber sheets developed in 12% w/v aqueous trichloroacetic acid. The ITLC sheets were cut into two halves, and each half was counted in a gamma counter or a dose calibrator. The radiolabeled annexin V-galactose conjugate precipitated at the origin, and any non-protein-bound radioactivity migrated with the solvent front in this solvent system. Tc-99m-annexin V-galactose conjugate (300-350 µg) solution was diluted and stored in PBS containing bovine serum albumin (commercially available from Sigma Chemical Co.) at a final concentration of 15-20 mg/ml PBS prior to intravenous injection.
E. Blood Clearance of Tc-99m-Annexin V and of Tc-99m-Annexin V-Galactose. An animal model, as previously described in Example III, was employed to evaluate blood clearance of the molecules. For N=20 swine, the blood clearance (Figure 2) is biphasic (biexponential):

| | | |
|---|---|---|
| %ID/g | a = 0.0389 | t1/2 a = 9.6 min |
| %ID/g | b = 0.0300 | t1/2 b = 46 min |

a + b = 0.0689, therefore 57% (0.0389/0.0689) of the injected dose/gram of blood is cleared in the faster a phase and 43% of the ID/g is cleared in the slower b phase.
Blood clearance of Tc-99m-annexin V-galactose in the swine (Figure 2) is also biphasic (biexponential):

| | | |
|---|---|---|
| %ID/g | a = 0.0313 | t1/2 a = 3.5 min |
| %ID/g | b = 0.0045 | t1/2 b = 160 min |

a + b = 0.0358, therefore 87% (0.0313/0.0358) of the injected dose/gram of blood is cleared very quickly in the early a phase. This more rapid clearance reduces the background radioactivity in the blood compartment, i.e., the noise, and therefore improves the signal to noise ratio. Consequently, thrombus imaging can be achieved at shorter time points.

## Claims

1. An annexin-containing conjugate suitable for radiolabeling with a diagnostic imaging agent comprising:
an annexin;
one or more hexose residues; and an N₂S₂ or N₃S compound capable of complexing a radionuclide, wherein the compound is associated with the annexin.

2. An annexin-containing conjugate of claim 1 wherein the annexin is annexin V.

3. An annexin-containing conjugate of claim 1 of the following formula: 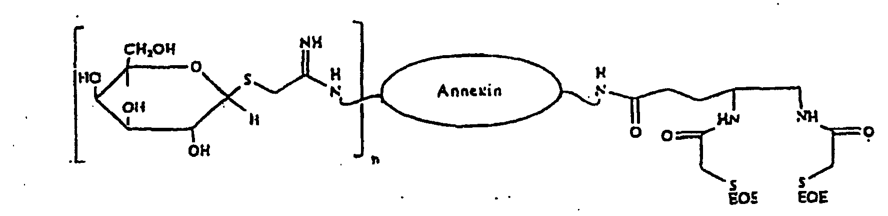 wherein n is one or more,

4. An annexin-containing conjugate of claim 3 wherein n ranges from 1 to 5 galactose residues.

5. A radiolabeled annexin-hexose suitable for imaging vascular thrombi, which radiolabeled annexin-hexose comprises.
an annexin;
one or more hexose residues;
an N₂S₂ or N₃S compound capable of complexing a radionuclide, wherein the compound is associated with the annexin; and a diagnostic radionuclide complexed by the compound.

6. A radiolabeled annexin-hexose of claim 5 wherein the annexin is annexin V.

7. A radiolabeled annexin-hexose of claim 5 of the following formula: 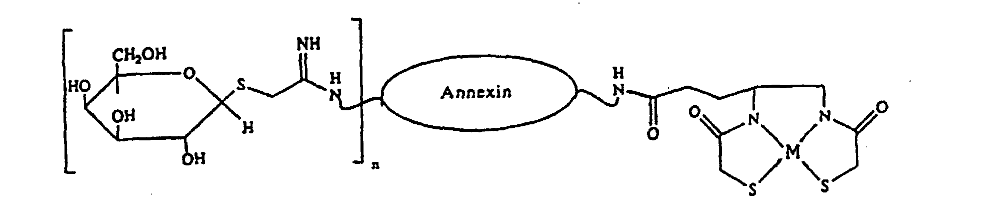 wherein n is one or more and M is a radionuclide.

8. A radiolabeled annexin-hexose of claim 5, wherein the radionuclide is selected from the group consisting of Tc-99m, Re-186, Re-188, Pd-100, Bi-212, Pb-212, Pd-109 and Cu-67.

9. A radiolabeled annexin-hexose of claim 5, wherein the radionuclide is Tc-99m.

10. A radiolabeled annexin-hexose of claim 5 wherein n ranges from 1 to 5 galactose residues.

## Patentansprüche

1. Annexin enthaltendes Konjugat, das für radioaktive Markierung mit einem diagnostischen bildgebenden Mittel geeignet ist, umfassend:
ein Annexin;
einen oder mehrere Hexosereste; und eine N₂S₂- oder N₃S-Verbindung, die ein Radionuklid komplexieren kann, wobei die Verbindung mit dem Annexin verbunden ist.

2. Annexin enthaltendes Konjugat nach Anspruch 1, **dadurch gekennzeichnet, daß** das Annexin Annexin V ist.

3. Annexin enthaltendes Konjugat nach Anspruch 1 der folgenden Formel: 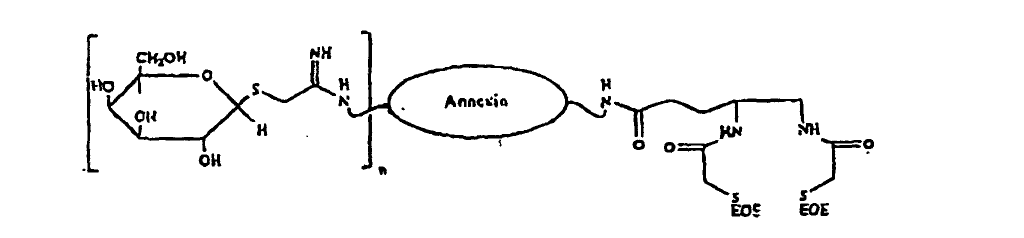 worin n eins oder mehr ist.

4. Annexin enthaltendes Konjugat nach Anspruch 3, **dadurch gekennzeichnet, daß** n im Bereich von 1 bis 5 Galactoseresten liegt.

5. Radioaktiv markierte Annexin-Hexose, die geeignet ist zur Abbildung von Gefäßthromben, wobei die radioaktiv markierte Annexin-Hexose umfaßt:
ein Annexin;
einen oder mehrere Hexosereste;
eine N₂S₂- oder N₃S-Verbindung, die ein Radionuklid komplexieren kann, wobei die Verbindung mit dem Annexin verbunden ist; und ein diagnostisches Radionuklid, das von der Verbindung komplexiert ist.

6. Radioaktiv markierte Annexin-Hexose nach Anspruch 5, **dadurch gekennzeichnet, daß** das Annexin Annexin V ist.

7. Radioaktiv markierte Annexin-Hexose nach Anspruch 5 der folgenden Formel: 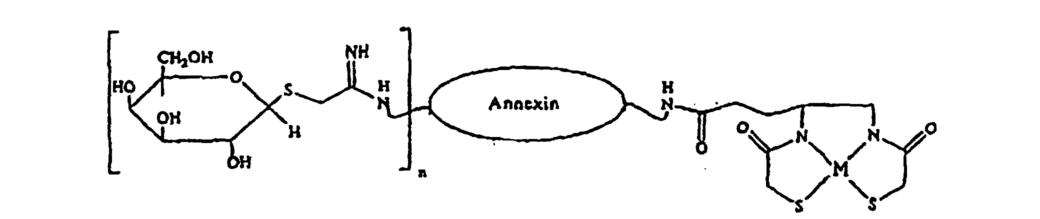 worin n eins oder mehr ist und M ein Radionuklid ist.

8. Radioaktiv markierte Annexin-Hexose nach Anspruch 5, **dadurch gekennzeichnet, daß** das Radionuklid ausgewählt ist aus der Gruppe, die aus Tc-99m, Re-186, Re-188, Pd-100, Bi-212, Pb-212, Pd-109 und Cu-67 besteht.

9. Radioaktiv markierte Annexin-Hexose nach Anspruch 5, **dadurch gekennzeichnet, daß** das Radionuklid Tc-99m ist.

10. Radioaktiv markierte Annexin-Hexose nach Anspruch 5, **dadurch gekennzeichnet, daß** n in einem Bereich von 1 bis 5 Galactoseresten liegt.

## Revendications

1. Conjugué contenant une annexine, apte au radio-marquage avec un agent d'imagerie de diagnostic, comprenant :
une annexine ;
un ou plusieurs résidus hexose ; et un composé de formule N₂S₂ ou N₃S apte à la complexation d'un radionucléide, le composé étant associé à l'annexine.

2. Conjugué contenant une annexine suivant la revendication 1, dans lequel l'annexine est l'annexine V.

3. Conjugué contenant une annexine suivant la revendication 1, répondant à la formule suivante : 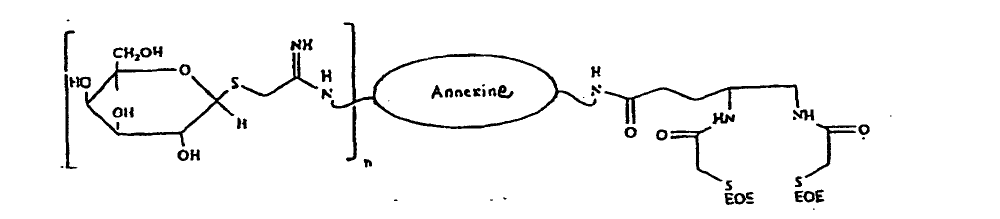 dans laquelle n est égal ou supérieur à un.

4. Conjugué contenant une annexine suivant la revendication 1, dans lequel n représente 1 à 5 résidus galactose.

5. Annexine-galactose radiomarqué apte à l'imagerie de thrombus vasculaires, annexine-galactose radiomarqué qui comprend :
une annexine ;
un ou plusieurs résidus hexose ;
un composé de formule N₂S₂ ou N₃S apte à la complexation d'un radionucléide, le composé étant associé à l'annexine ; et un radionucléide de diagnostic complexé par le composé.

6. Annexine-galactose radiomarqué suivant la revendication 5, dans lequel l'annexine est l'annexine V.

7. Annexine-galactose radiomarqué suivant la revendication 5, répondant à la formule : 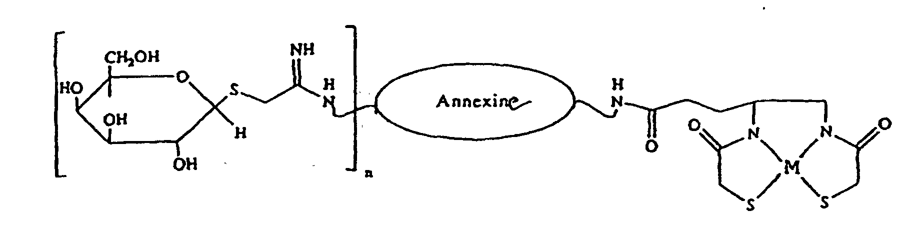 dans laquelle n est égal ou supérieur à un et M représente un radionucléide.

8. Annexine-galactose radiomarqué suivant la revendication 5, dans lequel le radionucléide est choisi dans le groupe consistant en Tc-99m, Re-186, Re-188, Pd-100, Bi-212, Pb-212, Pd-109 et Cu-67.

9. Annexine-galactose radiomarqué suivant la revendication 5, dans laquelle le radionucléide consiste en Tc-99m.

10. Annexine-galactose radiomarqué suivant la revendication 5, dans laquelle n représente 1 à 5 résidus galactose.
